# EUROPEAN PATENT APPLICATION

(11) **EP 3 919 440 A1**
(43) Date of publication of application: **08.12.2021**
(21) Application number: 20749632.4
(22) Date of filing: 09.01.2020
(51) Int. Cl.: C01B 25/37, C09C 1/36, A61K 8/00, A61K 8/29

(54) **TITANIUM PHOSPHATE POWDER AND WHITE PIGMENT FOR COSMETIC PREPARATIONS**

(30) Priority: 29.01.2019 JP 2019013228
(71) Applicant: Fujimi Incorporated, Kiyosu-shi, Aichi 452-8502 (JP)
(72) Inventor: IWAKUNI Mayumi, Kiyosu-shi, Aichi 452-8502 (JP); ASHITAKA Keiji, Kiyosu-shi, Aichi 452-8502 (JP); MIWA Naoya, Kiyosu-shi, Aichi 452-8502 (JP)
(74) Representative: Trautmann, Martin Tobias
(86) International application number: PCT/JP2020/000525
(87) International publication number: WO 2020/158332

(57) **Abstract**

A titanium phosphate powder suitable as a raw material for cosmetic products and a raw material for coating materials is provided. A titanium phosphate powder according to one aspect of the present invention is a titanium phosphate powder including plate-shaped crystal particles of titanium phosphate, in which a modified layer is present on a surface of the plate-shaped crystal particles.

## Description

### Technical Field

The present invention relates to a titanium phosphate powder and a white pigment for cosmetic preparations.

### Background Art

PTL 1 discloses a titanium phosphate powder composed of plate-shaped crystal particles of titanium phosphate. In this titanium phosphate powder, an average thickness of the plate-shaped crystal particles is 0.01 µm or more and less than 0.10 µm, and an aspect ratio, which is a value obtained by dividing an average primary particle diameter of the plate-shaped crystal particles by the average thickness, is 5 or more. Titanium phosphate composed of such thin plate-shaped crystal particles has favorable slippery properties between particles, and is therefore suitable as an additive added to cosmetic preparations and the like (for example, a white pigment for cosmetic preparations) and as a pigment added to coating materials.

### Citation List

### Patent Literature

PTL 1: WO 2018/180797

### Summary of Invention

### Technical Problem

However, because titanium phosphate is hydrophilic, it has low dispersion stability in an organic solvent, and even in a case where it is formed into a dispersion liquid, there is a high probability that particles will aggregate and a particle size of a titanium phosphate powder will increase. That is, as it is, it is not possible to obtain a dispersion liquid of titanium phosphate with a hydrophobic liquid as a solvent while exhibiting favorable physical properties of the titanium phosphate powder of PTL 1. An organic solvent dispersion liquid of titanium phosphate is useful as a base liquid for coating materials and cosmetic products.

An object of the present invention is to provide a titanium phosphate powder suitable as a raw material for cosmetic products and a raw material for coating materials.

### Solution to Problem

A titanium phosphate powder according to one aspect of the present invention is a titanium phosphate powder comprising plate-shaped crystal particles of titanium phosphate, in which a modified layer is present on a surface of the plate-shaped crystal particles.

### Advantageous Effects of Invention

According to the present invention, it is possible to provide a titanium phosphate powder suitable as a raw material for cosmetic products and a raw material for coating materials.

### Brief Description of Drawings

FIG. 1 is a graph illustrating a relationship between a phosphorus concentration [P] of a raw material (a mixture of titanium(IV) sulfate or titanyl sulfate and phosphoric acid) and an average thickness of hexagonal plate-shaped crystal particles of the obtained titanium phosphate, for titanium phosphate powders (before hydrophobization treatment) of sample Nos. 1 to 12 obtained in Examples;
FIG. 2 is a graph illustrating a relationship between a phosphorus concentration [P] of the raw material (the mixture of titanium(IV) sulfate or titanyl sulfate and phosphoric acid) and an average primary particle diameter of the hexagonal plate-shaped crystal particles of the obtained titanium phosphate, for the titanium phosphate powders (before hydrophobization treatment) of sample Nos. 1 to 12 obtained in Examples;
FIG. 3 is a graph illustrating a relationship between an average thickness and an average primary particle diameter of the hexagonal plate-shaped crystal particles of the titanium phosphate, for the titanium phosphate powders (before hydrophobization treatment) of sample Nos. 1 to 12 obtained in Examples;
FIG. 4 is a graph illustrating a relationship between an average primary particle diameter and an aspect ratio of the hexagonal plate-shaped crystal particles of the titanium phosphate, for the titanium phosphate powders (before hydrophobization treatment) of sample Nos. 1 to 12 obtained in Examples;
FIG. 5 is a graph illustrating a relationship between a phosphorus concentration [P] of the raw material and an average primary particle diameter of the hexagonal plate-shaped crystal particles of the obtained titanium phosphate, for the titanium phosphate powders (before hydrophobization treatment) obtained with a constant concentration ratio [P]/[Ti] of the raw material (the mixture of titanium(IV) sulfate or titanyl sulfate and phosphoric acid) in Examples;
FIG. 6 is an SEM image of the titanium phosphate powder of sample No. 2 (before hydrophobization treatment);
FIG. 7 is an SEM image of the titanium phosphate powder of sample No. 11 (before hydrophobization treatment);
FIG. 8 is an SEM image of the titanium phosphate powder of sample No. 13 (before hydrophobization treatment);
FIG. 9 is an SEM image of the titanium phosphate powder of sample No. 14 (before hydrophobization treatment); and
FIG. 10 is an SEM image of the titanium phosphate powder of sample No. 15 (before hydrophobization treatment).

### Description of Embodiments

An embodiment of the present invention will be described in detail. The following embodiment shows an example of the present invention, and the present invention is not limited to the present embodiment. Furthermore, various changes or enhancements can be added to the following embodiment, and embodiments to which changes or enhancements are added may also be included in the present invention.

A titanium phosphate powder of the present embodiment is composed of plate-shaped crystal particles of titanium phosphate, in which a modified layer is present on a surface of the plate-shaped crystal particles. The modified layer in the titanium phosphate powder of the present embodiment is a hydrophobized layer. Examples of hydrophobized layers other than the hydrophobized layer include a dispersion stability improving layer, an adherability improving layer, a wettability improving layer, a hydrophilicity improving layer, an adsorptiveness improving layer, a catalytically active layer, an aggregating property improving layer, and the like.

Examples of methods of providing the modified layer on the surface of the plate-shaped crystal particle made of titanium phosphate include a mechanochemical reaction method, a particle composite method, an etching method, an electrophoresis method, a physical vapor deposition method, a chemical vapor deposition method, a sol-gel formation reaction method, a precipitation method, a metallic soap treatment method, and the like.

An average thickness of the plate-shaped crystal particles constituting the titanium phosphate powder of the present embodiment is 0.01 µm or more and 4 µm or less, and an aspect ratio, which is a value obtained by dividing an average primary particle diameter of the plate-shaped crystal particles by the average thickness, is 5 or more. Furthermore, in the titanium phosphate powder of the present embodiment, an average primary particle diameter of the plate-shaped crystal particles of titanium phosphate is 0.05 µm or more and 20 µm or less, and the plate-shaped crystal particles of titanium phosphate are hexagonal plate-shaped crystal particles.

The titanium phosphate powder of the present embodiment is a titanium phosphate powder composed of the plate-shaped crystal particles having a high aspect ratio, which is a value obtained by dividing a particle diameter by a thickness, and having an average thickness of 4 µm or less. Therefore, slippery properties between the particles of titanium phosphate is favorable. Furthermore, since the hydrophobized layer is present on the surface of the plate-shaped crystal particle in the titanium phosphate powder of the present embodiment, it is possible to obtain a dispersion liquid of titanium phosphate with a hydrophobic liquid as a solvent.

Accordingly, the titanium phosphate powder of the present embodiment is suitable as an additive added to cosmetic preparations such as sunscreen cosmetic preparations and as a pigment added to coating materials. Furthermore, the titanium phosphate powder of the present embodiment is also suitable as a white pigment for cosmetic preparations.

The titanium phosphate powder of the present embodiment can be obtained by reacting a raw material containing titanium and phosphorus by a hydrothermal synthesis method to manufacture plate-shaped crystal particles of titanium phosphate, and thereafter, subjecting the obtained plate-shaped crystal particles to hydrophobization treatment by a sol-gel formation reaction method or a metallic soap treatment method.

As the raw material used in the hydrothermal synthesis method, it is possible to use a mixture of titanium(IV) sulfate or titanyl sulfate and phosphoric acid. By using titanium sulfate as a titanium source, it becomes easy to obtain the plate-shaped crystal particles of titanium phosphate which are thin and have a high aspect ratio.

Reaction conditions for the hydrothermal synthesis method are not particularly limited, but a reaction temperature is, for example, 100°C or higher and 160°C or lower.

When the reaction temperature is 160°C or lower, it is possible to use a reaction vessel, which is made of a glass lining material and is general purpose equipment, when manufacturing the titanium phosphate powder, and thereby manufacturing costs can be reduced. Furthermore, when the temperature is 160°C or lower, manufacturing can be performed in a Class 1 pressure vessel (pressure of 1 MPa or less). Furthermore, when the temperature is 160°C or lower, a concentration of chemicals at the time of manufacturing can be set under a wider range of conditions.

Meanwhile, when the reaction temperature is 100°C or higher, it is possible to easily obtain the plate-shaped crystal particles of titanium phosphate which have high crystallinity, and furthermore, because a viscosity of a product is low, it is also possible to manufacture a titanium phosphate powder with simple manufacturing equipment.

However, when the reaction temperature is 100°C or lower, there is a concern of a slight reduction in crystallinity of the plate-shaped crystal particles of titanium phosphate, and furthermore, a viscosity of a product is slightly increased, there is a concern of this increase affecting the design of manufacturing equipment. Therefore, the reaction temperature is more preferably 110°C or higher and 160°C or lower. Within a range of 100°C or higher and 160°C or lower, there is no great difference in crystallinity of the plate-shaped crystal particles of titanium phosphate.

Furthermore, a ratio [P]/[Ti] of a molar concentration [P] of phosphorus to a molar concentration [Ti] of titanium as a raw material is, for example, 3 or more and 21 or less. When the ratio [P]/[Ti] is 3 or more, or preferably 5 or more, the plate-shaped crystal particles of titanium phosphate are easily generated. Meanwhile, in a case where a concentration of titanium as a raw material is the same, as the ratio [P]/[Ti] becomes larger, an average primary particle diameter of the plate-shaped crystal particles of titanium phosphate tends to become smaller, but even when the ratio [P]/[Ti] exceeds 21, an average primary particle diameter is not further reduced and is almost constant.

Furthermore, the concentration of titanium as a raw material is, for example, 0.05 mol/L or more and 1.0 mol/L or less. In a case where the ratio [P]/[Ti] is the same, as the concentration of titanium as a raw material becomes higher, an average primary particle diameter and an average secondary particle diameter of the plate-shaped crystal particles of titanium phosphate tend to become smaller. Furthermore, by increasing the concentration of titanium as a raw material, manufacturing costs can be reduced. Accordingly, the concentration of titanium as a raw material is preferably 0.05 mol/L or more, and is more preferably 0.2 mol/L or more.

However, when the concentration of titanium as a raw material is excessively high, there is a concern of an increase in viscosity of a product and a decrease in uniformity of the product. Therefore, the concentration of titanium as a raw material is preferably 1.0 mol/L or less, and is more preferably 0.6 mol/L or less.

### Examples

Examples will be shown below, and the present invention will be described in more detail.

### [Manufacture of titanium phosphate powder before hydrophobization treatment]

Titanium(IV) sulfate or titanyl sulfate and phosphoric acid were reacted by a hydrothermal synthesis method, and thereby titanium phosphate powders (before hydrophobization treatment) of sample No. 1 to No. 15 were manufactured. For each of the samples, the following conditions for hydrothermal synthesis were changed as shown in Table 1: a concentration (molar concentration) of titanium [Ti] as a raw material (a mixture of titanium(IV) sulfate or titanyl sulfate and phosphoric acid), a concentration (molar concentration) of phosphorus [P] as a raw material, a ratio [P]/[Ti] of both of the concentrations, a reaction temperature, and a reaction time.

Specifically, first, titanium(IV) sulfate or titanyl sulfate as a titanium source and phosphoric acid as a phosphorus source were mixed to obtain a mixture. Titanium(IV) sulfate was used as a titanium source for sample No. 1 to No. 10 and No. 13. Titanyl sulfate was used as a titanium source for sample No.11, No.12, No.14, and No.15. In addition, 85% phosphoric acid (an aqueous solution of phosphoric acid at a concentration of 85% by mass) was used for sample No. 1 to No. 3 and No. 10 to No. 15, and 75% phosphoric acid (an aqueous solution of phosphoric acid at a concentration of 75% by mass) was used for sample No. 4 to No. 9.

Next, the obtained mixture was heated to a predetermined temperature in a reaction vessel. At that time, the reaction vessel was not pressurized, and a pressure inside the vessel was set to a value naturally determined by the heating temperature (natural pressurization). After the reaction for a predetermined time, a slurry-like product was obtained in the vessel. The obtained product was cooled, washed with water, and dried. Thereby, titanium phosphate powders (before hydrophobization treatment) were obtained.

An average primary particle diameter and an average thickness of each of the obtained titanium phosphate powders No. 1 to No. 15 (before hydrophobization treatment) were measured, and an aspect ratio was calculated from these numerical values. A measurement value of the average primary particle diameter is a value obtained by analyzing an image obtained by a scanning electron microscope using image analysis software "Mac-View ver.4" manufactured by Mountech Co., Ltd. These values are shown in Table 1 together with the conditions for hydrothermal synthesis.

**[Table 1]**

| No. | Conditions for hydrothermal synthesis | | | | | Titanium phosphate powder (before hydrophobization treatment) | | |
|---|---|---|---|---|---|---|---|---|
| | Concentration of titanium (mol/L) | Concentration of phosphorus (mol/L) | Ratio of concentrations [P]/[Ti] | Reaction temperature (°C) | Reaction time (h) | Average primary particle diameter (µm) | Average thickness (µm) | Aspect ratio |
| 1 | 0.30 | 3.96 | 13 | 160 | 5 | 0.35 | 0.048 | 7 |
| 2 | 0.30 | 3.96 | 13 | 110 | 5 | 0.24 | 0.017 | 14 |
| 3 | 0.30 | 3.96 | 13 | 100 | 5 | 0.21 | 0.019 | 11 |
| 4 | 0.22 | 3.27 | 15 | 160 | 5 | 1.33 | 0.099 | 13 |
| 5 | 0.22 | 4.55 | 21 | 160 | 5 | 0.24 | 0.026 | 9 |
| 6 | 0.26 | 4.29 | 17 | 130 | 5 | 0.19 | 0.025 | 8 |
| 7 | 0.39 | 5.24 | 13 | 110 | 5 | 0.12 | 0.014 | 9 |
| 8 | 0.45 | 6.08 | 14 | 110 | 5 | 0.078 | 0.012 | 7 |
| 9 | 0.58 | 7.75 | 13 | 110 | 5 | 0.072 | 0.014 | 5 |
| 10 | 0.60 | 3.32 | 6 | 110 | 5 | 0.886 | 0.084 | 11 |
| 11 | 0.30 | 3.22 | 11 | 130 | 5 | 3.04 | 0.27 | 11 |
| 12 | 0.24 | 3.31 | 14 | 130 | 10 | 10.35 | 0.63 | 16 |
| 13 | 0.60 | 2.58 | 4 | 110 | 5 | - | - | - |
| 14 | 0.30 | 3.93 | 13 | 90 | 5 | 0.22 | 0.030 | 7 |
| 15 | 0.30 | 3.25 | 11 | 130 | 5 | 5.76 | 0.66 | 9 |

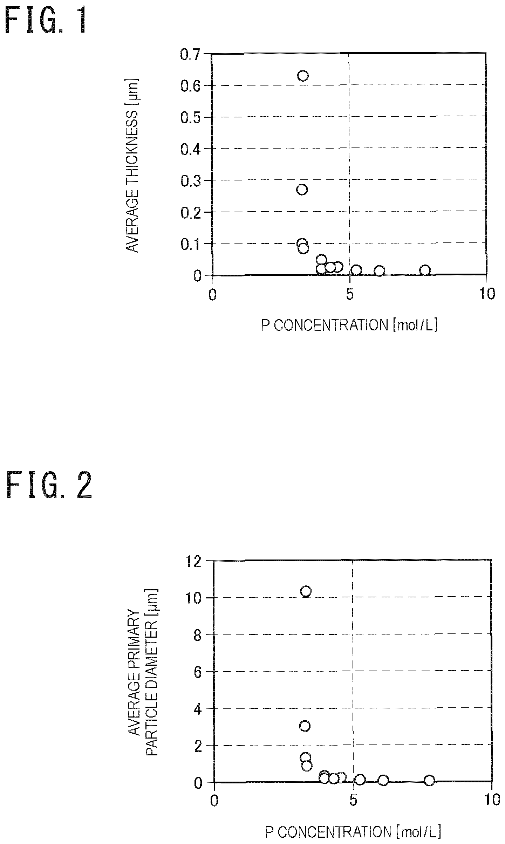

Furthermore, regarding the titanium phosphate powders of sample Nos. 1 to 12 (before hydrophobization treatment), a graph of FIG. 1 illustrates a relationship between a concentration of phosphorus [P] as a raw material and an average thickness of hexagonal plate-shaped crystal particles of the obtained of titanium phosphate. Regarding the titanium phosphate powders of sample Nos. 1 to 12 (before hydrophobization treatment), a graph of FIG. 2 illustrates a relationship between a concentration of phosphorus [P] as a raw material and an average primary particle diameter of the hexagonal plate-shaped crystal particles of the obtained of titanium phosphate.

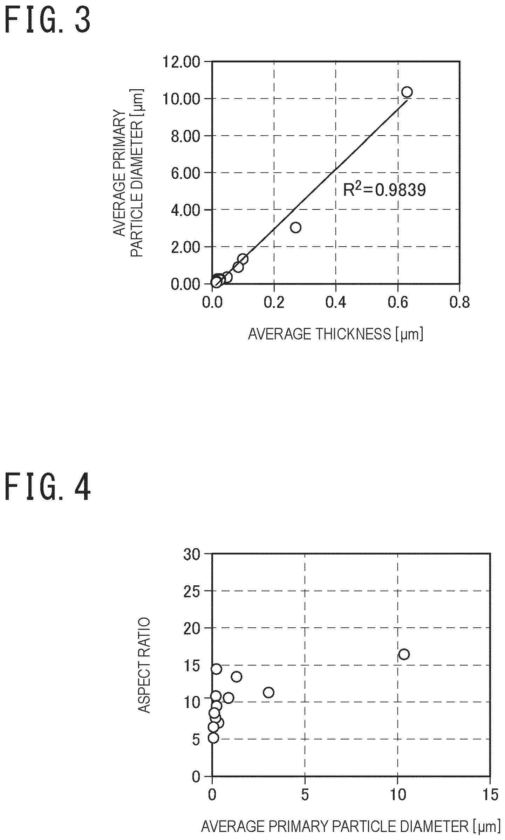

Furthermore, regarding the titanium phosphate powders of sample Nos. 1 to 12 (before hydrophobization treatment), a graph of FIG. 3 illustrates a relationship between an average thickness and the average primary particle diameter of the hexagonal plate-shaped crystal particles of the obtained of titanium phosphate. Regarding the titanium phosphate powders of sample Nos. 1 to 12 (before hydrophobization treatment), a graph of FIG. 4 illustrates a relationship between the average primary particle diameter and an aspect ratio of the hexagonal plate-shaped crystal particles of the obtained of titanium phosphate.

### (Regarding concentration of titanium)

A titanium phosphate powder was manufactured by a hydrothermal synthesis method with a constant (16.5) ratio [P]/[Ti] of concentrations of the raw materials, and 0.22 mol/L or 0.26 mol/L of a concentration of titanium. A reaction temperature was 110°C, 120°C, 130°C, or 160°C.

As a result, when the concentration of titanium was higher, an average primary particle diameter of plate-shaped crystal particles of titanium phosphate was smaller.

Next, a titanium phosphate powder was manufactured by a hydrothermal synthesis method with a constant (13.4) ratio [P]/[Ti] of concentrations of the raw materials, and 0.39 mol/L, 0.45 mol/L, 0.52 mol/L, or 0.58 mol/L of a concentration of titanium. A reaction temperature was constant (110°C).

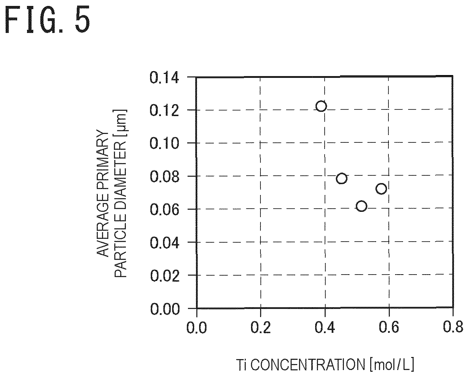

As a result, it was found that when a concentration of titanium was higher, manufacturing costs could be more reduced. Furthermore, a graph of FIG. 5 illustrates a relationship between the concentration of titanium and an average primary particle diameter of plate-shaped crystal particles of the obtained titanium phosphate. As can be seen from this graph, when the concentration of titanium was higher, an average primary particle diameter of plate-shaped crystal particles of titanium phosphate was smaller.

Based on these results, it was found that it is possible to control an average primary particle diameter of the plate-shaped crystal particles of titanium phosphate to a desired size by decreasing a concentration of phosphorus and increasing a concentration of titanium.

### (Regarding concentration of phosphorus)

An influence of a concentration of phosphorus in a case where a concentration of titanium was increased (for example, 0.4 mol/L or more) was examined. A titanium phosphate powder was manufactured by a hydrothermal synthesis method with 0.22 mol/L, 0.41 mol/L, or 0.60 mol/L of a concentration of titanium while changing a ratio [P]/[Ti] of concentrations and a concentration of phosphorus to various values. A reaction temperature was 160°C in a case where a concentration of titanium was 0.22 mol/L, and was 110°C in a case where a concentration of titanium was 0.41 mol/L and 0.60 mol/L.

As a result, with all of the concentrations of titanium, when a concentration of phosphorus was 2.6 mol/L or less, titanium phosphate was decreased in crystallinity and thus was not formed into plate-shaped crystal particles, whereas when a concentration of phosphorus was 3.3 mol/L or more, plate-shaped crystal particles of titanium phosphate were produced. Furthermore, in the same manner as in a case where a concentration of titanium was low (for example, 0.2 mol/L), there was a tendency for an average primary particle diameter of plate-shaped crystal particles of titanium phosphate to become smaller when a concentration of phosphorus was higher.

In addition, from the examination in which a concentration of titanium was constant (0.60 mol/L) and a concentration of phosphorus was changed to various values (3.3, 4.09, and 4.91 mol/L), it was found that an average primary particle diameter of the plate-shaped crystal particles of titanium phosphate changed according to the concentration of phosphorus.

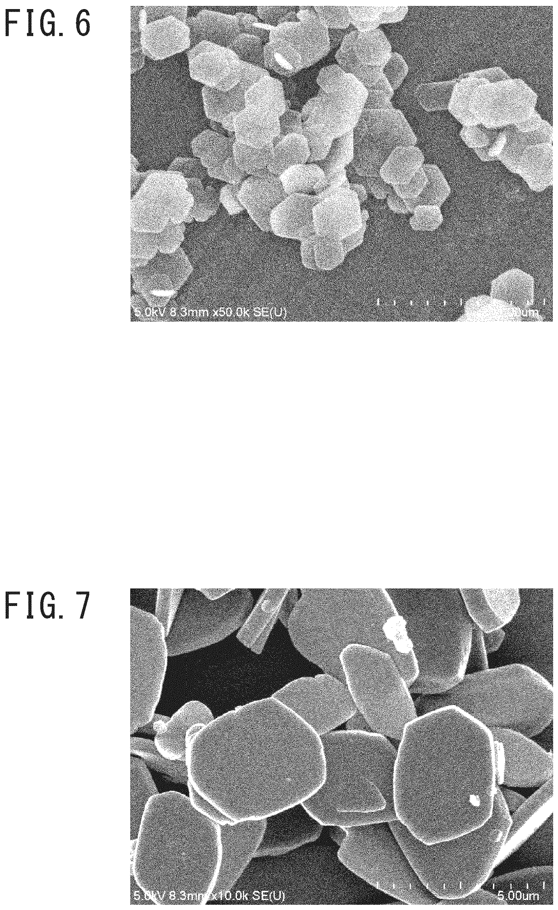

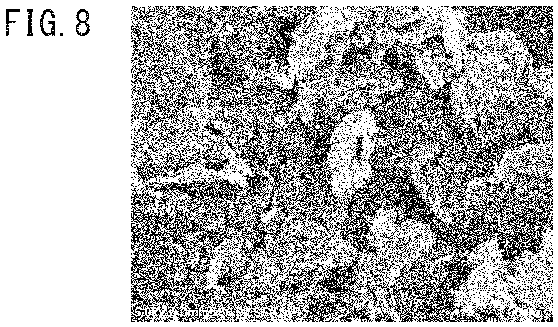

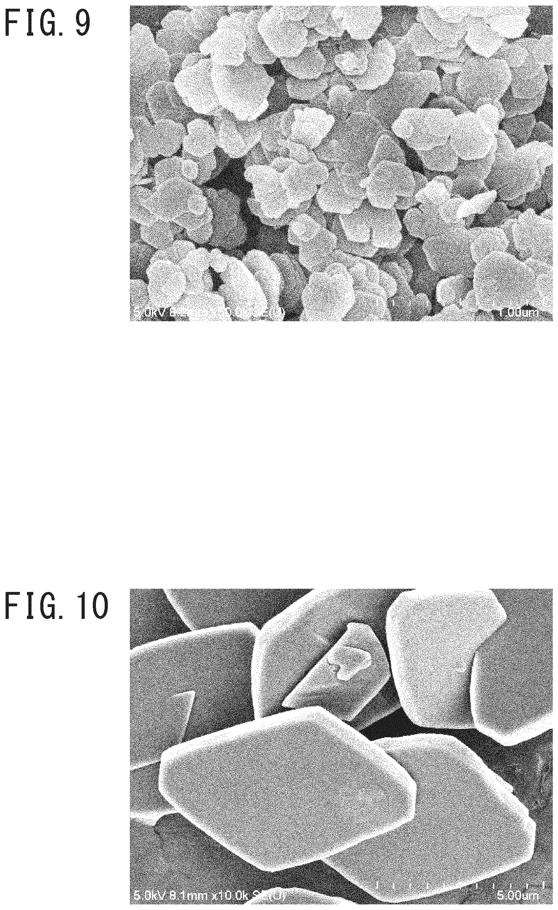

FIG. 6 is an SEM image of the titanium phosphate powder of No. 2 (before hydrophobization treatment), FIG. 7 is an SEM image of the titanium phosphate powder of No. 11 (before hydrophobization treatment), and FIG. 8 is an SEM image of the titanium phosphate powder of No. 13 (before hydrophobization treatment). Furthermore, FIG. 9 is an SEM image of the titanium phosphate powder of No. 14 (before hydrophobization treatment), and FIG. 10 is an SEM image of the titanium phosphate powder of No. 15 (before hydrophobization treatment). As can be seen from FIGS. 6 to 10, particles constituting the titanium phosphate powders of No. 2, No. 11, No. 14, and No. 15 (before hydrophobization treatment) were in a hexagonal plate shape, whereas particles constituting the titanium phosphate powder of No. 13 (before hydrophobization treatment) were in a rod shape instead of a plate shape.

### [Hydrophobization treatment 1]

The titanium phosphate powder of No. 11 (before hydrophobization treatment) was subjected to hydrophobization treatment by the following method (sol-gel formation reaction method, dry-type), and thereby a titanium phosphate powder in which a hydrophobized layer (modified layer) was present on the surface of plate-shaped crystal particles of titanium phosphate was obtained.

First, an ethanol acetate solution with an acetic acid concentration of 0.05% by mass was prepared. Next, 3 g of triethoxy(octyl)silane was added into 3 g of this ethanol acetate solution. The mixed solution was stirred at room temperature for 3 hours, and thereby a triethoxy(octyl)silane diluent solution was obtained. Next, the obtained triethoxy(octyl)silane diluent solution was added to a dry powder of the titanium phosphate powder of No. 11 (before hydrophobization treatment) in an amount of 3% by mass with respect to a mass of this dry powder, and mixed to obtain a mixture. Next, the mixture was left to stand at room temperature for 12 hours and then heated at 180°C for 5 hours.

When the titanium phosphate powder that had been hydrophobized in this manner was added to water, the titanium phosphate powder was separated into two layers and became a state of floating on water even under stirring. Therefore, it could be confirmed that the hydrophobized layer was present on the surface of the plate-shaped crystal particles of titanium phosphate.

### [Hydrophobization treatment 2]

The titanium phosphate powder of No. 14 (before hydrophobization treatment) was subjected to hydrophobization treatment by the following method (sol-gel formation reaction method, wet-type), and thereby a titanium phosphate powder in which a hydrophobized layer (modified layer) was present on the surface of plate-shaped crystal particles of titanium phosphate was obtained.

First, an ethanol acetate solution with an acetic acid concentration of 0.5% by mass was prepared. Next, 0.2 g of triethoxy(octyl)silane was added into 9.8 g of this ethanol acetate solution. The mixed solution was stirred at room temperature for 24 hours, and thereby a triethoxy(octyl)silane diluent solution was obtained.

Next, 8.87 g of the obtained triethoxy(octyl)silane diluent solution was added to a 20 g of a slurry containing 10% by mass of the titanium phosphate powder of No. 14 (before hydrophobization treatment), and mixed to obtain a mixture. Next, the mixture was stirred at room temperature for 24 hours. Thereafter, solid-liquid separation was performed. A solid content was washed with water, dried at 100°C, and then heated at 150°C for 3 hours.

When the titanium phosphate powder that had been hydrophobized in this manner was added to water, the titanium phosphate powder was separated into two layers and became a state of floating on water even under stirring. Furthermore, when the titanium phosphate powder was added into isododecane as a hydrophobic solvent and stirred, the powder became a dispersed state. Based on these results, it could be confirmed that the hydrophobized layer was present on the surface of the plate-shaped crystal particles of titanium phosphate. When the titanium phosphate powder of No. 14 before hydrophobization treatment was added into water and stirred, it became a dispersed state. When this titanium phosphate powder was added into isododecane, the titanium phosphate powder was separated into two layers even under stirring and became a state of sinking underneath.

### [Hydrophobization treatment 3]

The titanium phosphate powder of No. 15 (before hydrophobization treatment) was subjected to hydrophobization treatment by the following method (sol-gel formation reaction method, wet-type), and thereby a titanium phosphate powder in which a hydrophobized layer (modified layer) was present on the surface of plate-shaped crystal particles of titanium phosphate was obtained.

First, an ethanol acetate solution with an acetic acid concentration of 0.5% by mass was prepared. Next, 0.1 g of triethoxy(octyl)silane was added into 9.9 g of this ethanol acetate solution. The mixed solution was stirred at room temperature for 24 hours, and thereby a triethoxy(octyl)silane diluent solution was obtained.

Next, 3.5 g of the obtained triethoxy(octyl)silane diluent solution was added to a 50 g of a slurry containing 10% by mass of the titanium phosphate powder of No. 15 (before hydrophobization treatment), and mixed to obtain a mixture. Next, the mixture was stirred at room temperature for 1 hour. Thereafter, solid-liquid separation was performed. A solid content was washed with water, dried at 100°C, and then heated at 150°C for 3 hours.

When the titanium phosphate powder that had been hydrophobized in this manner was added to water, the titanium phosphate powder was separated into two layers and became a state of floating on water even under stirring. Furthermore, when the titanium phosphate powder was added into isododecane as a hydrophobic solvent and stirred, the powder became a dispersed state. Based on these results, it could be confirmed that the hydrophobized layer was present on the surface of the plate-shaped crystal particles of titanium phosphate.

When the titanium phosphate powder of No. 15 before hydrophobization treatment was added into water and stirred, it became a dispersed state. When this titanium phosphate powder was added into isododecane, the titanium phosphate powder was separated into two layers even under stirring and became a state of sinking underneath.

### [Hydrophobization treatment 4]

The titanium phosphate powder of No. 15 (before hydrophobization treatment) was subjected to hydrophobization treatment by the following method (metallic soap treatment method, double decomposition method), and thereby a titanium phosphate powder in which a hydrophobized layer (modified layer) was present on the surface of plate-shaped crystal particles of titanium phosphate was obtained.

First, 50 g of a slurry containing 20% by mass of the titanium phosphate powder of No. 15 (before hydrophobization treatment) was heated to 70°C. Next, 1 g of a sodium stearate powder was gradually added into this slurry and dissolved. Next, 2.16 g of an aqueous solution of a 40% by mass magnesium sulfate heptahydrate was added dropwise to this slurry. Next, the slurry obtained after the aqueous solution was added dropwise thereto was stirred for 10 minutes. Until this point, a temperature of the slurry was maintained at 70°C.

Next, the slurry was cooled to room temperature. Solid-liquid separation was performed, and a solid content was washed with water. Next, the solid content was dried at 110°C for 4 hours. As a result, a soap layer made of magnesium stearate was formed on the surface of the titanium phosphate powder.

When the titanium phosphate powder that had been hydrophobized in this manner was added to water, the titanium phosphate powder was separated into two layers and became a state of floating on water even under stirring. Furthermore, when the titanium phosphate powder was added into isododecane as a hydrophobic solvent and stirred, the powder became a dispersed state. Based on these results, it could be confirmed that the hydrophobized layer was present on the surface of the plate-shaped crystal particles of titanium phosphate.

## Claims

1. A titanium phosphate powder comprising:
plate-shaped crystal particles of titanium phosphate,
wherein a modified layer is present on a surface of the plate-shaped crystal particles.

2. The titanium phosphate powder according to claim 1, wherein the modified layer is a hydrophobized layer.

3. The titanium phosphate powder according to claim 1 or 2,
wherein an average thickness of the plate-shaped crystal particles is 0.01 µm or more and 4 µm or less, and
an aspect ratio, which is a value obtained by dividing an average primary particle diameter of the plate-shaped crystal particles by the average thickness, is 5 or more.

4. The titanium phosphate powder according to claim 3, wherein the average primary particle diameter is 0.05 µm or more and 20 µm or less.

5. The titanium phosphate powder according to any one of claims 1 to 4, wherein the plate-shaped crystal particles are hexagonal plate-shaped crystal particles.

6. A white pigment for cosmetic preparations comprising the titanium phosphate powder according to any one of claims 1 to 5.
